(19) Europäisches Patentamt
European Patent Office
Office européen des brevets

(11) Numéro de publication: **0 062 596**
**B1**

# (12) FASCICULE DE BREVET EUROPEEN

(45) Date de publication du fascicule du brevet: **03.07.85**

(51) Int. Cl.⁴: **C 07 C 93/06**, C 07 D 319/20, A 61 K 31/135, A 61 K 31/335

(21) Numéro de dépôt: **82450006.0**

(22) Date de dépôt: **01.04.82**

(54) **Nouveaux dérivés d'aminoéthoxy-4 isopropyl-5 méthyl-2 phénol, leur méthode de préparation et leur emploi en tant que médicaments.**

(30) Priorité: **06.04.81 FR 8106832**
**04.03.82 FR 8203795**
**04.03.82 FR 8203796**

(43) Date de publication de la demande:
**13.10.82 Bulletin 82/41**

(45) Mention de la délivrance du brevet:
**03.07.85 Bulletin 85/27**

(84) Etats contractants désignés:
**BE CH DE GB IT LI LU NL**

(56) Documents cités:
**FR - A - 1 405 494**
**FR - A - 2 278 329**
**FR - M - 1 310**
**GB - A - 745 070**
**GB - A - 922 600**

(73) Titulaire: **SOCIETE CORTIAL S.A., 7 rue de l'Armorique, F-75015 Paris (FR)**

(72) Inventeur: **Courtiol, Christian, 10 rue Armand Calmon, F-33600 Pessac (FR)**
Inventeur: **Creuzet, Marie-Hélène, Résidence Jardin de Gambetta T 3, F-33000 Bordeaux (FR)**
Inventeur: **Feniou, Claude, 5 rue du Général Guillomat, F-33600 Pessac (FR)**
Inventeur: **Prat, Gisèle, Hameau de Noailles Villa 18, F-33400 Talence (FR)**
Inventeur: **Pontagnier, Henri, 21 rue Edouard Vaillant, F-33600 Pessac (FR)**

(74) Mandataire: **Tajan, Marie-Thérèse, LABORATOIRES SARGET Avenue du Président JF Kennedy, F-33701 Mérignac (FR)**

**EP 0 062 596 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux produits à activité alphabloquante et antiagrégante plaquettaire, leur méthode de synthèse, les préparations pharmaceutiques les contenant et leur application en thérapeutique.

Ces nouveaux produits ont pour formule générale

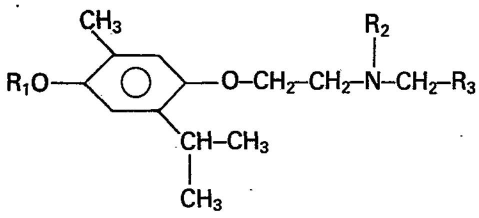

(I)

formule dans laquelle

$R_1$ = H, $-CH_3$, $-COCH_3$
$R_2$ = H, $CH_3$

$R_3$ =

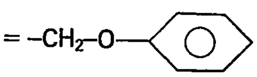

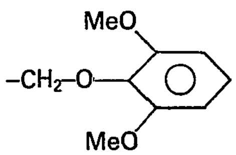

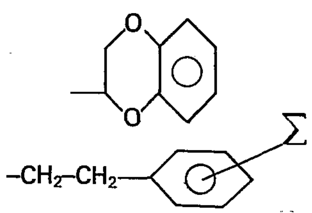

avec = H ou un ou plusieurs substituants tels que OH,$OCH_3$, halogène.

Ils peuvent être sous forme de base lire ou de sels pharmaceutiquement compatibles tels que les sels d'addition d'acides minéraux (par exemple acides chlorhydrique, bromhydrique, sulfurique, phosphorique) ou organiques (par exemple acides citrique, méthanesulfonique, camphosulfonique).

La classe des médicaments à activité alpha bloquante comprend des dérivés agissant sur des récepteurs présynaptiques (ou récepteurs alpha2) tels que la yohimbine, des dérivés agissant spécifiquement sur les récepteurs post synaptiques (ou récepteurs alphal) tels que le moxisylyte ou la prazosine et des dérivés agissant à la fois sur les récepteurs pré- et post synaptiques tels que la phentolamine. Les dérivés à activité alpha-bloquante post synaptique spécifique sont d'un très grand intérêt car ils entrainent une diminution des résistances périphériques artérielles et veineuses sans présenter les effets secondaires indésirables manifestés par les dérivés à action mixte alpha1 et alpha2, (intolérance cardiaque, tachycardie). Actuellement les dérivés alpha1-bloquants tels que la prazosine sont surtout employés dans le traitement de l'hypertension, de l'insuffisance cardiaque et des troubles vasomoteurs des extrémités.

Compte tenu de la présence de récepteurs alpha dans un très grand nombre d'organes, on peut envisager l'emploi des alpha-bloquants dans le traitement de l'angor pour supprimer le spasme coronarien, dans la prévention des phénomènes thromboemboliques, dans le traitement des crises d'asthme, en combinaison avec un béta-stimulant.

Les produits de la présente invention dérivent de la formule du moxisylyte

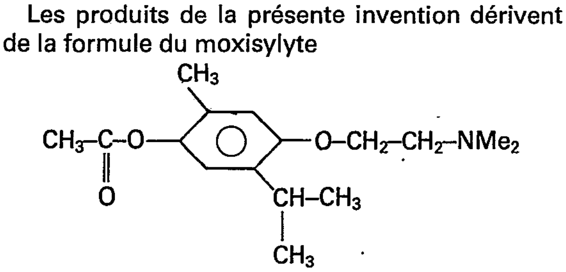

Ils s'en différencient de façon principale par la présence sur l'atome d'azote d'une deuxième chaine contenant un cycle aromatique.

H. Kapur et D.R. Mottram (J. Pharm. Pharmacol., 1975, 27, 295 et Biochem. Pharmacol., 1978, 27, 1879) ont décrit des produits voisins et par exemple le WB4101

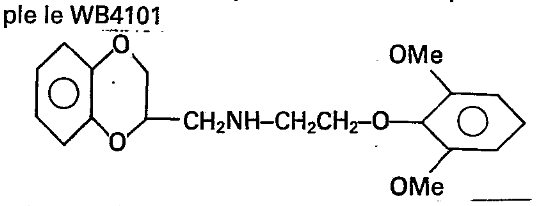

Les produits de la présente invention présentent par rapport aux produits alpha bloquants connus une activité alpha1 bloquante souvent de longue durée jointe à une faible toxicité. Ils présentent de plus une activité antiagrégante plaquettaire permettant leur emploi dans la prévention ou le traitement des thromboses.

Les produits de la présente invention sont préparés à partir de l'isopropyl-5-méthyl-2 N-méthylaminoéthoxy-4 phénol par l'intermédiaire des produits de formule I tels que $R_1$ = H et $R_2$ = $CH_3$. Ce phénol réagit avec le chlorure de l'acide $R_3COOH$, dans lequel $R_3$ a la signification indiquée plus haut, pour former

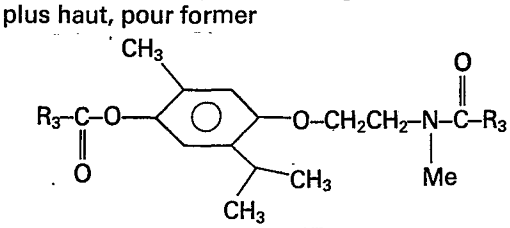

Ce dérivé est réduit par $LiA1H_4$ pour obtenir

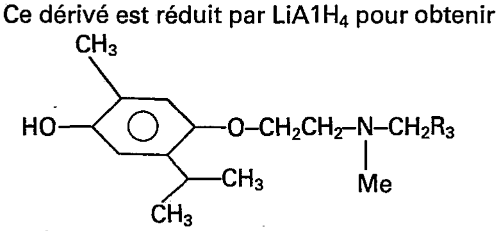

Les dérivés tels que $R_1$ = $CH_3$ ou $COCH_3$ sont obtenus par les réactions classiques de méthylation et d'acétylation des OH phénoliques.

Les dérivés tels que $R_2$ = H sont obtenus en deux temps par réaction avec BrCN conduisant à

un cyanamide suivie d'une réduction par $LiA1H_4$ conduisant au produit désiré.

L'invention va être décrite plus précisément dans les exemples suivants sans toutefois que ceux-ci ne limitent sa portée.

Exemple 1

Synthèse de l'isopropyl-5 méthyl-2 (N-méthyl N-phénoxyéthyl aminoéthoxy)-4 phénol (formule I avec $R_1$ = H, $R_2$ = $CH_3$,

$R_3$ = 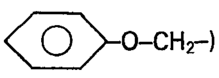

On dissout 20,5 g d'isopropyl-5 méthyl-2 N-méthylaminoéthoxy-4 phénol dans un mélange de 200 cm³ de benzène et 27 cm³ de triéthylamine. On ajoute goutte à goutte 36 g de chlorure d'acide phénoxyacétique en solution dans 100 cm³ de benzène. On chauffe le mélange réactionnel pendant deux heures. Le benzène est éliminé par évaporation et le résidu est repris par une solution d'HCl 2N. Le résidu est extrait par 2 fois 100 cm³ de chloroforme. La phase chloroformique est lavée, séchée. Le chloroforme est éliminé par évaporation. On obtient ainsi 44 g de proudit de formule

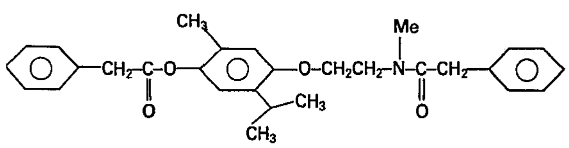

12,5 g de $LiA1H_4$ sont mis en suspension dans 500 cm³ d'éther éthylique. On ajoute goutte à goutte 79 g du produit précédemment préparé en solution dans 350 cm³ d'éther éthylique. On chauffe 3 h au reflux puis on refroidit. L'excès de $LiA1H_4$ est détruit par de l'eau saturée en $Na_2SO_4$. La solution est acidifiée par une solution d'acide sulfurique. Après décantation la phase aqueuse est alcalinisée jusqu'au pH 9 par une solution d'ammoniaque. On obtient ainsi 48 g du produit de l'exemple 1.

Exemple 2

Synthèse de l'((isopropyl-2 méthoxy-4 méthyl-5) phénoxy)-2 N-méthyl N-phénoxyéthyl éthylamine (formule I avec $R_1$ = $CH_3$, $R_2$ = $CH_3$,

$R_3$ = 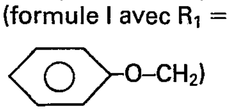

On ajoute sous agitation à température ambiante de petites quantités de diazométhane en solution éthérée à une solution de 21 g du produit de l'exemple 1 dans 200 cm³ d'éther éthylique. On contrôle par de l'acide acétique la présence de diazométhane en excès. L'évolution de la réaction est suivie en HPLC analytique dans les conditions suivantes (colonne µ Bondapack C18, éluant méthanol 65 cm³/eau 35 cm³/Pic B7 1 dose/litre, débit 2 ml/mn, détection UV 280 nm, k' du produit de départ = 0,9, k' du produit final = 3). Quand la réaction de méthylation est terminée l'excès de diazométhane est détruit en ajoutant petit à petit de l'acide acétique. Après purification on obtient 15 g du produit de l'exemple 2.

Exemple 3

Synthèse du chlorhydrate de l'((isopropyl-2 méthoxy-4 méthyl-5) phénoxy)-2 N-méthyl N-phénoxyéthyl éthylamine ou COR 2809.

5 g du produit de l'exemple 2 sont dissous dans 100 cm³ d'éther éthylique. Après refroidissement on y fait barboter HCl gazeux. Après filtration le chlorhydrate est lavé à l'éther. On obtient ainsi 5,2 g de produit de PF 97°C.

Exemple 4

Synthèse de l'((isopropyl-2 méthoxy-4 méthyl-5) phénoxy)-2 N-phénoxyéthyl éthylamine (formule I avec $R_1$ = $CH_3$, $R_2$ = H,

$R_3$ = 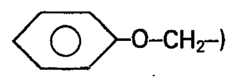

A température ambiante on ajoute goutte à goutte 11,2 g du produit de l'exemple 2 en solution dans 200 cm³ de benzène à une solution de 3,5 g de BrCN dans 10 cm³ de benzène. La réaction est suivie en HPLC analytique dans les conditions suivantes (colonne µ Bondapack C18, éluant méthanol 65 cm³/eau 35 cm³/Pic B7, 1 dose/litre, débit 2 cm³/mn, détection UV 280 nm, k' du produit initial 3, k' du produit final 6,5). Quand la réaction est totale le benzène est éliminé par évaporation au rotavapor et le résidu est repris dans 15 cm³ d'HCl 2N. L'insoluble est extrait par de l'éther éthylique. La phase éthérée est lavée, séchée et évaporée. On obtient ainsi 7 g du dérivé

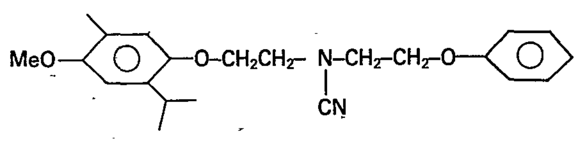

1,5 g de $LiA1H_4$ sont mis en suspension dans un mélange de 100 cm³ d'éther éthylique et 50 cm³ de tétrahydrofuranne. On ajoute goutte à goutte 7 g du cyanamide précédemment préparé en solution dans 40 cm³ d'éther éthylique et 10 cm³ de tétrahydrofuranne. Le mélange est chauffé au reflux pendant cinq heures. Après refroidissement l'excès de $LiA1H_4$ est détruit par une solution aqueuse saturée en $Na_2SO_4$. On acidifie avec une solution d'acide sulfurique; on décante. Les solvants de la phase organique sont éliminés par évaporation. Le résidu visqueux obtenu est trituré plusieurs fois dans de l'éther éthylique avant d'être repris par une solution d'ammoniaque. L'insoluble est extrait par de l'éther éthylique. Après lavage et séchage on élimine l'éther par évaporation. On obtient ainsi 2,5 g du produit de l'exemple 4.

Exemple 5

Synthèse du chlorhydrate de l'(isopropyl-2 méthoxy-4 méthyl-5) phénoxy-2 N-phénoxyéthyl éthylamine ou COR 28010.

2,5 g du produit de l'exemple 4 sont dissous dans 50 cm³ d'éther. On fait barboter de l'acide chlorhydrique gazeux dans cette solution. On obtient ainsi 1,7 g du produit de l'exemple 5 sous forme d'une poudre blanche.

Exemple 6

Synthèse de (N-(diméthoxy-2,6- phénoxyéthyl) N-méthylaminoéthoxy)-4 isopropyl-5 méthyl-2 phénol (formule I avec $R_1$ = H, $R_2$ = $CH_3$,

$R_3$ = 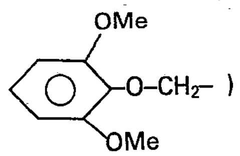

On dissout 23 g d'isopropyl-5 méthyl-2 N-méthylaminoéthoxy-4 phénol dans un mélange de 200 cm³ de benzène et 30 cm³ de triéthylamine. On ajoute goutte à goutte 47 g du chlorure de l'acide diméthoxy-2,6 phénoxyacétique en solution dans 100 cm³ de benzène. Le mélange réactionnel est ensuite chauffé à 50°C pendant deux heures. Le benzène est éliminé par évaporation au rotavapor et le résidu est repris par une solution d'HCl 2N. L'insoluble est extrait par deux fois 150 cm³ de chloroforme. La phase chloroformique est lavée et séchée et le chloroforme est éliminé par évaporation. On obtient ainsi 63 g de

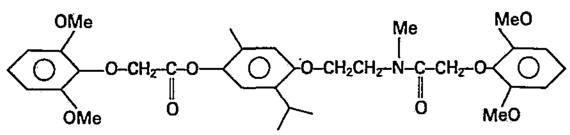

8,4 g de $LiA1H_4$ sont mis en suspension dans un mélange de 500 cm³ d'éther éthylique et 200 cm³ de tétrahydrofuranne. On ajoute goutte à goutte 62 g du produit précédemment préparé en solution dans 100 cm³ de tétrahydrofuranne. Le mélange est chauffé pendant trois heures au reflux puis l'excès de $LiA1H_4$ est détruit, après refroidissement, à l'aide d'eau saturée en $Na_2SO_4$. On acidifie par une solution d'acide sulfurique; on décante. Le solvant de la phase organique est éliminé par évaporation. Le résidu est trituré plusieurs fois dans de l'éther éthylique avant d'être repris par une solution d'ammoniaque. Le précipité est extrait avec 2 fois 250 cm³ de chloroforme. La phase chloroformée est lavée et séchée. Le chloroforme est éliminé par évaporation, en on obtient 35 g du produit de l'exemple 6.

Exemple 7

Synthèse du chlorhydrate du (N-(diméthoxy-2,6 phénoxyéthyl) N-méthylaminoéthoxy)-4 isopropyl-5 méthyl-2 phénol ou COR 2822.

10 g du produit de l'exemple 6 sont dissous dans un mélange de 200 cm³ d'éther éthylique et de 50 cm³ de chloroforme. 50 cm³ d'éther éthylique saturés par de l'acide chlorhydrique gazeux sont ajoutés à cette solution. Le précipité formé est filtré et lavé à l'éther éthylique. On obtient 10 g du produit de l'exemple 7. PF = 161°C.

Exemple 8

Synthèse de la N-(diméthoxy-2,6 phénoxyéthyl) N-méthyl ((isopropyl-2 méthoxy-4 méthyl-5) phénoxy)-2 éthylamine (formule I avec $R_1$ = $CH_3$, $R_2$ = $CH_3$,

$R_3$ = 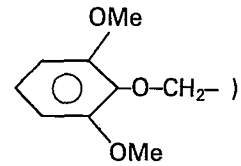

On ajoute à une solution de 26 g du produit de l'exemple 6 dans 200 cm³ d'éther, par petites fractions et sous agitation, du diazométhane en solution éthérée. L'excès de diazométhane est contrôlé par l'acide acétique. Quand la réaction est totale le diazométhane en excès est détruit par l'addition d'acide acétique. L'éther est éliminé par évaporation. Le produit est ensuite purifié par chromatographie sur colonne de silice dans les conditions suivantes: 16 g de produit brut sont mis en solution dans un minimum de toluène. On utilise une colonne contenant 200 g de silice Merck Kieselgur 60 dans le toluène. L'élution est réalisée par deux litres de toluène puis un mélange 99/1 V/V de toluène/méthanol. On obtient ainsi 7 g du produit de l'exemple 8.

Exemple 9

Synthèse du chlorhydrate de la N-(diméthoxy-2,6 phénoxyéthyl) N-méthyl ((isopropyl-2 méthoxy-4 méthyl-5) phénoxy)-2 éthylamine ou COR 2811.

On dissout 7 g du produit de l'exemple 8 dans 100 cm³ d'éther éthylique. Après refroidissement on fait barboter de l'acide chlorhydrique gazeux. On filtre et on lave avec un peu d'éther éthylique froid. On obtient 7 g du produit de l'exemple 9. PF = 97°C.

Exemple 10

Synthèse du (N-(diméthoxy-2,6 phénoxyéthyl) aminoéthoxy)-4 isopropyl-5 méthyl-2 phénol (produit de formule I avec $R_1$ = H, $R_2$ = H,

$R_3$ = 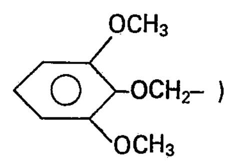

A une solution de 8 g de BrCN dans 110 cm³ de benzène on ajoute goutte à goutte à température ambiante une solution de 20 g de (N-(diméthoxy-2,6 phénoxyéthyl) N-méthylaminoéthoxy)-4 isopropyl-5 méthyl-2 phénol dans 80 cm³ de benzène. Le mélange est chauffé à 50°C pendant trois heures. Le benzène est éliminé par évaporation puis le résidu est purifié par extraction à chaud dans l'éther éthylique. L'éther est ensuite éliminé par évaporation et l'on obtient 10 g du produit de formule

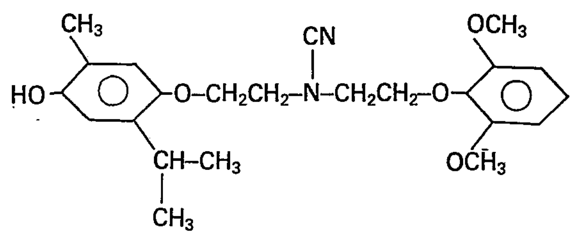

Ce dérivé est contrôlé en chromatographie sur couches minces sur plaques de silice dans un éluant composé de 90% de chloroforme et 10% de méthanol. 10g de ce produit en solution dans 100cm³ de tétrahydrofuranne sont ajoutés goutte à goutte à une suspension de 1,9g de $LiA1H_4$ dans 100cm³ d'éther. Le mélange est ensuite chauffé au reflux pendant cinq heures. Après refroidissement l'excès de $LiA1H_4$ est détruit par de l'eau saturée en $Na_2SO_4$. Le mélange est traité selon la méthode décrite dans l'exemple 4 pour obtenir le produit de l'exemple 10.

Exemple 11

Synthèse du chlorhydrate du (N-(diméthoxy-2,6 phénoxyéthyl) aminoéthoxy)-4 isopropyl-5 méthyl-2 phénol ou COR 2825.

3g du produit de l'exemple 10 sont mis en solution dans 100cm³ d'éther éthylique. On fait barboter de l'acide chlorhydrique gazeux dans cette solution puis après filtration le précipité formé est lavé avec de l'éther froid. On obtient ainsi le produit de l'exemple 11. PF = 118°C.

Exemple 12

Synthèse de la N-((hydroxy-4 isopropyl-2 méthyl-5) phénoxyéthyl) N-méthyl benzodioxanne-1,4 méthylamine-2 (formule I avec $R_1$ = H, $R_2 = CH_3$,

$R_3$ = 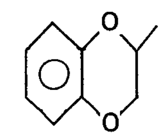 )

On dissout 40,1 g d'isopropyl-5 méthyl-2 N-méthylaminoéthoxy-4 phénol dans un mélange de 350cm³ de benzène et de 75cm³ de triéthylamine. On ajoute goutte à goutte 76,5g de chlorure de l'acide benzodioxanne-1,4 carboxylique-2 en solution dans 150cm³ de benzène. On chauffe ensuite le mélange réactionnel à 50°C pendant deux heures. Le benzène est éliminé par évaporation au Rotavapor et le résidu est repris par une solution 2N d'HCl. La partie insoluble est extraite par 2 fois 200cm³ de chloroforme. La phase chloroformique est lavée, séchée et le chloroforme est éliminé par évaporation. On obtient ainsi 99g du produit ayant la formule

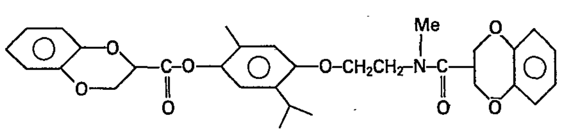

sous forme d'un solide beige.

On met en suspension 7,6g de $LiA1H_4$ dans un mélange de 500cm³ d'éther éthylique et 200cm³ de tétrahydrofuranne. On ajoute goutte à goutte 53g du produit précédemment préparé, mis en solution dans 100cm³ de tétrahydrofuranne. On chauffe 3 heures au reflux puis après refroissement on détruit l'excès de $LiA1H_4$ par de l'eau saturée en $Na_2SO_4$ avant d'acidifier par une solution d'acide sulfurique. La phase organique est séparée; le solvant est éliminé par évaporation. Le résidu visqueux est trituré plusieurs fois dans de l'éther éthylique avant d'être repris par une solution d'ammoniaque. L'insoluble est extrait par deux fois 150cm³ d'éther éthylique. La phase éthérée est lavée, séchée. L'éther est éliminé par évaporation. On obtient ainsi 33,5g du produit de l'exemple 12.

Exemple 13

Synthèse du chlorhydrate de la N-((hydroxy-4 isopropyl-2 méthyl-5) phénoxyéthyl) N-méthyl benzodioxanne-1,4 méthylamine-2 ou COR 28012.

5g du produit de l'exemple 12 sont dissous dans 100cm³ d'éther éthylique. On fait barboter de l'acide chlorhydrique gazeux dans cette solution. On filtre et on lave avec un peu d'éther froid le précipité formé.

Exemple 14

Synthèse du chlorhydrate de la N-((acétoxy-4 isopropyl-2 méthyl-5) phénoxyéthyl) N-méthyl benzodioxanne-1,4 méthylamine-2 (formule I avec $R_1 = COCH_3$, $R_2 = CH_3$,

$R_3$ = 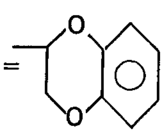 ;

chlorhydrate) ou COR 28013.

5g du produit de l'exemple 12 sont dissous dans un mélange de 25g d'anhydrique acétique et 3,16g de pyridine. Le mélange est porté au reflux pendant 1h30. La presque totalité de la pyridine et de l'anhydride acétique est éliminée sous vide. Le résidu est repris par quelques cm³ d'eau puis alcalinisé par l'ammoniaque. On extrait à l'éther. La phase organique est lavée, séchée puis le solvant est éliminé par évaporation. Le produit ainsi obtenu est dissout dans 70cm³ d'éther éthylique. On fait barboter dans cette solution de l'acide chlorhydrique gazeux. Le précipité est filtré puis lavé à l'éther éthylique. On obtient ainsi 3,5g du produit de l'exemple 14 sous forme d'un solide beige.

Exemple 15

Synthèse de la N-(hydroxy-4 isopropyl-2 méthyl-5) phénoxyéthyl) benzodioxanne-1,4 méthylamine-2 (formule I avec $R_1$ = H, $R_2$ = H,

$R_3$ = 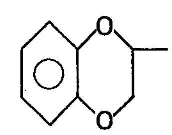 )

A température ambiante on ajoute goutte à goutte 56g du produit de l'exemple 12 en solution dans 80cm³ de benzène à une solution de BrCN dans 49cm³ de benzène. Le mélange est chauffé deux heures à 50°C. Le benzène est éliminé par évaporation au rotavapor, le résidu est repris dans 100cm³ d'HCl 2N. La phase insoluble est extraite par 2 fois 150cm³ de $CHCl_3$. Après lavage et séchage le chloroforme est éliminé par évaporation. On obtient 55g du dérivé

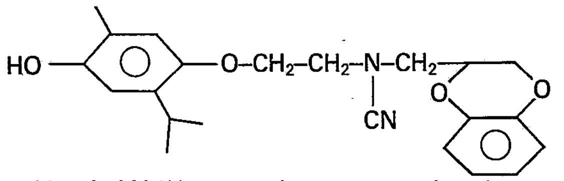

11 g de $LiA1H_4$ sont mis en suspension dans un mélange de 500 cm³ d'éther éthylique et 300 cm³ de tétrahydrofuranne. Les 55 g de cyanamide précédemment préparés mis en solution dans 100 cm³ de tétrahydrofuranne sont ajoutés goutte à goutte. Le mélange est chauffé au reflux pendant trois heures; après refroidissement l'excès de $LiA1H_4$ est détruit par de l'eau saturée en $Na_2SO_4$ avant d'acidifier par une solution d'acide sulfurique. Après décantation, les solvants de la phase organique sont éliminés par évaporation; le résidu visqueux est trituré plusieurs fois dans de l'éther éthylique avant d'être repris par une solution d'ammoniaque. La phase insoluble est extraite par 2 fois 150 cm³ d'éther éthylique. Après lavage et séchage l'éther est éliminé par évaporation. On obtient ainsi 30 g du produit de l'exemple 15.

Contrôle chromatographique sur couche mince.
Solvant $MeOH/CHCl_3$ 5/95 V/V
Révélateur $FeCl_3 - K_3Fe(CN)_6$
acide hexachloroplatinique

Exemple 16

Synthèse du chlorhydrate de la N-((hydroxy-4 isopropyl-2 méthyl-5) phénoxyéthyl) benzodioxanne-1,4 méthylamine ou COR 2814.

10 g du produit de l'exemple 15 sont dissous dans l'éther éthylique. On fait barboter de l'acide chlorhydrique gazeux. On filtre et on lave avec un peu d'éther éthylique froid. On obtient ainsi 10 g de produit de l'exemple 16.

Exemple 17

Synthèse de l'isopropyl-5 méthyl-2 (N-méthyl N-(phényl-3 propyl) aminoéthoxy)-4 phénol (formule I avec $R_1 = H$, $R_2 = CH_3$,

$R_3 =$ 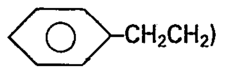

A une solution de 44,6 g d'isopropyl-5 méthyl-2 N-méthylaminoéthoxy-4 phénol dans un mélange de 350 cm³ de benzène et 80 cm³ de triéthylamine, on ajoute goutte à goutte 76 g de chlorure de l'acide phényl-3 propionique en solution dans 150 cm³ de benzène. Lorsque l'addition est terminée on chauffe au reflus pendant 4 heures. Après évaporation du solvant, le résidu est repris par une solution d'acide chlorhydrique 2N. Le résidu est extrait par deux fois 200 cm³ de chloroforme. La phase chloroformique est lavée à neutralité, séchée sur sulfate de sodium anhydre puis le solvant est évaporé. On obtient ainsi 95 g du produit de formule

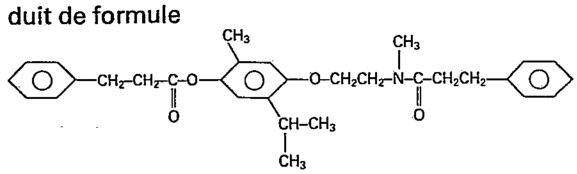

19,4 g de $LiA1H_4$ sont mis en suspension dans 750 cm³ d'éther éthylique anhydre. On ajoute goutte à goutte à cette suspension 95 g du produit précédemment préparé en solution dans 250 cm³ d'éther éthylique anhydre. Le mélange réactionnel est ensuite chauffé au reflux pendant six heures, puis refoidi dans un bain d'eau glacée. L'excès de $LiA1H_4$ est détruit par addition d'une solution aqueuse saturée en $Na_2SO_4$.

Le mélange réactionnel est versé dans 1,5 litre de glace pilée contenant 130 cm³ d'$H_2SO_4$ concentré. Après agitation et décantation on obtient 3 phases (une phase éthérée, une phase organique intermédiaire et une phase aqueuse). La phase organique intermédiaire est séparée par décantation puis alcalinisée par une solution d'ammoniaque. Cette solution alcaline est extraite par 2 fois 100 ml de chloroforme. La phase chloroformique est lavée à neutralité, séchée sur sulfate de sodium anhydre puis évaporée. On obtient ainsi 55,5 g du produit de l'exemple 17.

Exemple 18

Synthèse du chlorhydrate de l'isopropyl-5 méthyl-2 (N-méthyl N-(phényl-3 propyl) amino éthoxy)-4 phénol ou COR 2823.

50 cm³ d'éther éthylique préalablement saturé par de l'acide chlorhydrique gazeux sont ajoutés à une solution de 8 g du produit de l'exemple 17 en solution dans un mélange de 200 cm³ d'éther éthylique et 50 cm³ de chloroforme. Le précipité formé est filtré et lavé par l'éther éthylique. On obtient 8 g du produit de l'exemple 18.

Exemple 19

Synthèse de l'isopropyl-5 méthyl-2 (N-(phényl-3 propyl) aminoéthoxy)-4 phénol (formule I avec $R_1 = H$, $R_2 = H$,

$R_3 =$ 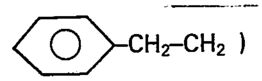

31 g du produit de l'exemple 17 en solution dans 80 cm³ de benzène sont ajoutés goutte à goutte à une solution de 10,6 g de BrCN dans 38 cm³ de benzène. Le mélange est chauffé à 50°C pendant trois heures. Après évaporation du benzène le résidu est repris par une solution diluée d'acide chlorhydrique. Cette solution acide est extraite par 2 fois 100 cm³ d'éther éthylique. La phase éthérée est lavée à neutralité, séchée sur sulfate de sodium anhydre puis évaporée. On obtient ainsi 20 g du dérivé

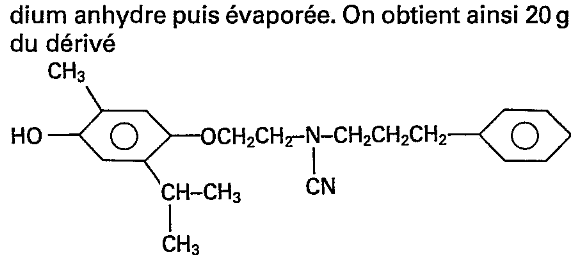

30 g de ce dérivé en solution dans 100 cm³ d'éther éthylique anhydre sont ajoutés goutte à goutte à une suspension de 6,5 g de $LiA1H_4$ dans 300 cm³ d'éther éthylique anhydre. Le mélange réactionnel est ensuite chauffé au reflux pendant huit heures, puis refroidi dans un bain d'eau gla-

cée. L'excès de $LiA1H_4$ est détruit par addition d'une solution aqueuse saturée par $Na_2SO_4$. Le mélange réactionnel est versé dans 0,5 litre de glace pilée contenant 25 cm³ d'acide sulfurique concentré. Après agitation et décantation, on obtient 3 phases; la phase organique intermédiaire est séparée par décantation puis alcalinisée par une solution d'ammoniaque. Cette solution alcaline est extraite par deux fois 100 cm³ d'éther éthylique. La phase éthérée est lavée à neutralité, séchée sur sulfate de sodium anhydre puis évaporée. On obtient ainsi 21,2 g du produit de l'exemple 19. Ce produit est purifié sur colonne de silice.

Exemple 20

Synthèse du chlorhydrate de l'isopropyl-5 méthyl-2 (N-(phényl-3 propyl) aminoéthoxy)-4 phénol ou COR 2821.

7,9 g du produit de l'exemple 19 sont dissous dans 100 cm³ d'éther éthylique. A cette solution est ajoutée une petite quantité d'éther éthylique saturée par de l'acide chlorhydrique gazeux. Le précipité est filtré et lavé à l'éther éthylique. On obtient ainsi 7,5 g du produit de l'exemple 20. Ce produit fond entre 60 et 80 °C.

Les deux tableaux ci-après donnent les spectres de RMN des produits décrits dans les exemples.

RMN des chlorhydrates, étalon interne TMS

| | | Protons | | | | | | |
|---|---|---|---|---|---|---|---|---|
| Produit nr | Solvant | | | | Protons aromatiques | OH | Protons $N^+$ | Autres protons |
| COR 2809 | $DMSOD_6$ | 6H; d;<br>1,2 ppm | 3H; s;<br>2,1 ppm | | 7H; mc;<br>6,6–7,6 ppm | | 1H; dôme;<br>12,0 ppm | 15H; mc;<br>2,7–4,8 ppm<br>dont $CH_3O$–<br>à 3,7 ppm |
| COR 2810 | $CDCl_3$ | 6H; d;<br>1,2 ppm | 3H; s;<br>2,2 ppm | | 7H; mc;<br>6,6–7,5 ppm | | 2H; dôme;<br>10,0 ppm | 12H; mc;<br>2,9–4,6 ppm<br>dont $CH_3O$–<br>à 3,8 ppm |
| COR 2811 | $CDCl_3$ | 6H; d;<br>1,2 ppm | 3H; s;<br>2,2 ppm | | 5H; mc;<br>6,4–7,2 ppm | | 1H; dôme;<br>12,7 ppm | 21H; mc;<br>3,1–4,7 ppm |
| COR 2812 | $DMSOD_6$ | 6H; d;<br>1,1 ppm | 3H; s;<br>2,1 ppm | | 6H; mc;<br>6,6–7,0 ppm | 1H; pe;<br>8,8 ppm | 1H; dôme;<br>11,7 ppm | 13H; mc;<br>2,7–5,3 ppm |
| COR 2813 | $CDCl_3$ | 6H; d;<br>1,1 ppm | 3H; s;<br>2,1 ppm | 3H; s;<br>2,3 ppm | 6H; mc;<br>6,7–7,0 ppm | | 1H; pic très étalé;<br>11–13 ppm | 13H; mc;<br>2,9–5,4 ppm |
| COR 2814 | $DMSOD_6$ | 6H; d;<br>1,1 ppm | 3H; s;<br>2,1 ppm | | 6H; mc;<br>6,6–7,0 ppm | 1H; dôme;<br>8,9 ppm | 2H; dôme;<br>9,9 ppm | 10H; mc;<br>3,0–5,1 ppm |
| COR 2821 | $CDCl_3$ +<br>4 gouttes<br>$DMSOD_6$ | 6H; d;<br>1,1 ppm | 3H; s;<br>2,1 ppm | | 2H; s; 6,6 et<br>6,7 ppm<br>(phénol)<br>5H; 7,1 ppm<br>($C_6H_5$) | 1H; dôme;<br>8,1 ppm | 2H; dôme;<br>9,6 ppm | 9H; mc;<br>1,9–3,5 ppm<br>2H; triplet;<br>4,2 ppm<br>$OCH_2$ |
| COR 2822 | $DMSOD_6$ | 6H; d;<br>1,1 ppm | 3H; s;<br>2,1 ppm | | 5H; mc;<br>6,5–7,2 ppm | 1H; dôme;<br>8,9 ppm | 1H; dôme;<br>11,4 ppm | 18H; mc;<br>2,9–4,6 ppm |
| COR 2823 | $CDCl_3$ +<br>5 gouttes<br>$DMSOD_6$ | 6H; d;<br>1,1 ppm | 3H; s;<br>2,1 ppm | | 2H; s;<br>6,5–6,7 ppm<br>(phénol)<br>5H; 7,1 ppm<br>($C_6H_5$) | 1H;<br>pic large;<br>8,2 ppm | 1H; dôme;<br>12,0 ppm | 12H; mc;<br>2,0–3,7 ppm<br>2H; triplet;<br>4,3 ppm<br>$OCH_2$ |
| COR 2825 | $CDCl_3$ +<br>$DMSOD_6$<br>50/50 | 6H; d;<br>1,1 ppm | 3H; s;<br>2,1 ppm | | 5H; mc;<br>6,4–7,2 ppm | 1H; pic très étalé;<br>7–10 ppm | 2H;<br>pic large;<br>9,3 ppm | 15H; mc;<br>3,0–4,5 ppm<br>dont $CH_3O$<br>à 3,8 ppm |

d = doublet s = singulet mc = massif complexe pe = pic étalé

RMN des bases libres, étalon interne TMS, solvant $CDCl_3$

| Produits des exemples | $C(CH_3)_2$ | CH | $NCH_3$ | OH | Protons aromatiques | Autres protons |
|---|---|---|---|---|---|---|
| 1 | 6H; d; 1,1 ppm | 3H; s; 2,2 ppm | 3H; s; 2,5 ppm | 1H; dôme; 5,5 ppm | 7H; mc; 6,5–7,4 ppm | 9H; mc; 2,8–4,3 ppm |
| 4 | 6H; d; 1,2 ppm | 3H; s; 2,2 ppm | | | 7H; mc; 6,5–7,5 ppm | 13H; mc; 2,7–4,3 ppm dont $CH_3O$ à 3,8 ppm |
| 6 | 6H; d; 1,1 ppm | 3H; s; 2,2 ppm | 3H; s; 2,5 ppm | 1H; dôme; 5,9 ppm | 5H; mc; 6,4–7,2 ppm | 15H; mc; 2,8–4,3 ppm dont $2CH_3O$ à 3,8 ppm |
| 12 | 6H; d; 1,1 ppm | 3H; s; 2,2 ppm | 3H; s; 2,4 ppm | 1H; dôme; 5,9 ppm | 6H; mc; 6,4–7,0 ppm | 10H; mc; 2,7–4,5 ppm |
| 15 | 6H; d; 1,2 ppm | 3H; s; 2,2 ppm | | dans le mc à 2,8–4,5 ppm | 6H; mc; 6,4–7,0 ppm | 12H; mc; 2,8–4,5 ppm |

d = doublet s = singulet mc = massif complexe

La toxicité des produits de la présente invention est déterminée chez la souris Swiss. Les animaux exempts d'organismes pathogènes spécifiques sont stabulés en salle climatisée 24 à 28 heures avant le début de l'expérimentation. Ils sont répartis en lots de 5 mâles et 5 femelles. Les substances sont administrées dans le véhicule approprié sous un volume équivalent à 0,1 ml pour 10 g de poids d'animal.

Les tableaux ci-après indiquent respectivement pour la voie orale et la voie intrapéritonéale les DL 50 déterminées pour chacun des produits par la méthode décrite par Cazin J.C. (Bull. Soc. Pharm. Lille, 1972, 4, 187) ou, dans le cas où la DL 50 n'a pas pu être déterminée, le pourcentage de mortalité induite par différentes doses.

Voie orale

| Produit | Solvant | Dose en mg/kg | Pourcentage de mortalité | DL 50 en mg/kg |
|---|---|---|---|---|
| COR 2809 | eau | 1000<br>2000 | 0<br>40 | |
| COR 2810 | gomme arabique | 1000 | 20 | |
| COR 2811 | gomme arabique | | | 546 (377–791) |
| COR 2812 | DMSO ½ | | | 1428 (1261–1617) |
| COR 2813 | DMSO ½ | 1000<br>2000 | 0<br>20 | |
| COR 2814 | DMSO ½ | | | 952 (790–1148) |
| COR 2821 | Tween | | | 358,6 (300,4–428,1) |
| COR 2822 | Tween | | | 316 (253–396) |
| COR 2823 | Tween | | | 615,3 (527,8–717,3) |
| Moxisylyte | gomme arabique | | | 225 (227–286) |

DMSO = diméthylsulfoxyde

Voie Intraperitoneale

| Produit | Solvant | DL 50 mg/kg |
|---|---|---|
| COR 2809 | DMSO ⅓ | 200 (188–212) |

Voie Intraperitoneale

| Produit | Solvant | DL 50 mg/kg |
|---|---|---|
| COR 2810 | eau | 81 (65–100) |

Voie Intraperitoneale

| Produit | Solvant | DL 50 mg/kg |
|---|---|---|
| COR 2811 | eau | 93 (72–120) |
| COR 2812 | DMSO ⅓ | 350 |
| COR 2813 | DMSO ⅓ | 600 (463–778) |
| COR 2814 | DMSO ⅓ | 174 (151–200) |
| COR 2821 | Tween | 84,6 (80,2–89,3) |
| COR 2822 | Tween | 66,2 (60,7–72,3) |
| COR 2823 | Tween | 154 (125,3–189,6) |
| Moxisylyte | Sérum physiologique | 73 (67–79) |

In vitro l'activité alpha-bloquante est déterminée par l'antogonisme des contractions de strips aortiques isolés de lapin induites par la noradrénaline employée à la concentration de $2.10^{-6}$ M/l. La technique employée est dérivée de celle de Furchgott & Bhadrakom (J. Pharmacol., 1953, 108, 129–43). L'agoniste est introduit dans le bain 30 secondes ou 14 mn après l'antagoniste. On donne dans le tableau ci-dessous les CI 50 exprimées en M/l c'est à dire les concentrations en produit alpha bloquant entrainant 50% d'inhibition de la contraction témoin induite par la noradrénaline.

| Produit | CI 50 M/l |
|---|---|
| COR 2809 | $2,6.10^{-4}$ |
| COR 2810 | $3,5.10^{-4}$ |
| COR 2811 | $2,16.10^{-5}$ * |
| COR 2812 | $9.10^{-5}$ |
| COR 2813 | $2,52.10^{-4}$ |
| COR 2814 | $6.10^{-6}$ |
| COR 2821 | $1,17.10^{-5}$ * |
| COR 2822 | $1,78.10^{-7}$ * |
| COR 2823 | $2,70.10^{-6}$ * |
| COR 2825 | $8,36.10^{-7}$ * |
| Moxisylyte | $1,7.10^{-5}$ |

* temps de contact 14 mn

In vivo l'activité alpha-bloquante est appréciée par la détermination de l'antagonisme de l'hypertension induite par la phényléphrine. Pour la voie intraveineuse on utilise des rats amyélés et bivagotomisés d'après la technique de J.S. Gillespie et T.C. Muir (Br. J. Pharmac. Chemother., 1967, 30, 78–87). Les alpha-bloquants sont administrés à la dose de 0,2, 1,6 et 3,2 mg/kg et le pourcentage d'activité inhibitrice maximale ainsi que le temps au bout duquel l'activité résiduelle est égale à 50% de l'activité maximale sont déterminés. Les résultats sont représentés dans le tableau ci-dessous.

| Produit | 0,2 mg/kg | | 1,6 mg/kg | | 3,2 mg/kg | |
|---|---|---|---|---|---|---|
| | % d'activité maximale | durée en mn | % d'activité maximale | durée en mn | % d'activité maximale | durée en mn |
| COR 2809 | | | 51,4 ± 5,10 | 4 mn 29 s | 74,7 ± 5,2 | 7 mn 20 s |
| COR 2810 | | | 66,3 ± 1,83 | 5 mn 33 s | 77,0 | 12 mn 44 s |
| COR 2811 | | | 61,8 ± 4,90 | > 60 mn | 74,60 ± 4,4 | 60 mn |
| COR 2812 | | | 59,1 ± 7,50 | 5 mn 53 s | 65,7 ± 4,5 | 6 mn 16 s |
| COR 2813 | | | 46,1 ± 6,01 | 5 mn 40 s | 61,1 ± 6,4 | 9 mn 9 s |
| COR 2814 | | | 92,0 ± 2,45 | 26 mn 2 s | 100,0 ± 0,0 | >60 mn |
| COR 2821 | | | 73,44 ± 6,99 | 50 mn | | |
| COR 2822 | 91,12 ± 3,46 | 35 mn | 112,48 ± 7,30 | 120 mn | | |
| COR 2823 | | | 81,09 ± 2,35 | 20 mn | | |
| COR 2825 | 70,20 ± 4,89 | 20 mn | 94,84 ± 2,22 | 120 mn | | |
| Moxisylyte | | | 83,1 ± 5,71 | 17 mn 20 s | 82,6 ± 4,54 | 18 mn 23 s |

Pour la voie orale on utilise des rats mâles vigiles chez lesquels des cathéters ont été implantés dans l'artère carotide et la veine jugulaire.

Le COR 2811 entraine à la dose de 5 mg/kg une activité maximale de 56,7 + 10,25%. Au bout de 60 minutes l'activité est encore de 30%. L'activité

antihypertensive est déterminée chez le rat spontanément hypertendu de souche Okamoto anesthésié au pentobarbital. Administré à la dose de 0,1 mg/kg par voie intraveineuse le produit COR 2811 fait passer la pression sanguine moyenne de 135 à 106 mn de mercure.

L'activité antiagrégante plaquettaire a été mise en évidence sur plaquettes humaines non lavées de sujet adulte normal étudiées dans leur milieu naturel (plasma riche en plaquettes). L'agent inhibiteur est testé en présence de 4 types d'agents agrégants (ADP, adrénaline, collagène, acide arachidonique) à diverses concentrations.

Dans chaque cas on établit une courbe dose réponse. Le pouvoir inhibiteur de la substance est caractérisé par la dose correspondant à 50% d'inhibition ou 150.

Le tableau ci-dessous indique pour certains produits de la présente invention les valeurs des 150 exprimées en M/l vis-à-vis des 4 agents agrégants et comparativement à la ticlopidine prise comme référence.

| Produit testé / Agent agrégant | formule I avec $R_1 = CH_3$ $R_2 = CH_3$ $R_3$ = 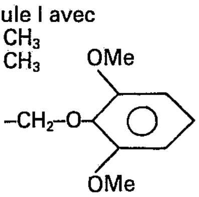 . HCl | formule I avec $R_1 = H$ $R_2 = H$ $R_3$ = 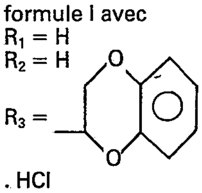 . HCl | Ticlopidine |
|---|---|---|---|
| ADP 1,25 μ M/l | $7.10^{-5}$ | $6.10^{-5}$ | $10^{-5}$ |
| ADP 2,5 μ M/l | $7,4.10^{-5}$ | $5,8.10^{-5}$ | $5.10^{-5}$ |
| adrénaline 1 μ M/l | $1,6.10^{-5}$ | $2,4.10^{-5}$ | $3,7.10^{-5}$ |
| adrénaline 5 μ M/l | $4.10^{-5}$ | $5.10^{-5}$ | $5,2.10^{-5}$ |
| collagène 50 μ g/ml | $> 10^{-4}$ | $5,2.10^{-5}$ | $> 10^{-4}$ |
| acide arachidonique $5.10^{-4}$ M/l | $> 10^{-4}$ | $1,5.10^{-5}$ | |
| $10^{-3}$ M/l | $> 10^{-4}$ | $5,4.10^{-5}$ | |

Compte tenu de leurs propriétés pharmacologiques se caractérisant par une activité alpha-bloquante et antihypertensive, les produits faisant l'objet de la présente invention peuvent être employés par exemple dans le traitement de l'hypertension artérielle (seuls ou en association avec un diurétique ou d'autres médicaments antihypertenseurs), dans le traitement des troubles vasculaires périphériques tels que l'acrocyanose et le syndrôme de Raynaud, dans le traitement de la cirse d'asthme (seuls ou en association avec un betastimulant), dans le traitement du glaucome.

Compte tenu de leurs propriétés antiagrégantes plaquettaires les produits selon la présente invention pourront être employés en traitement au long cours, seuls ou associés, dans la prophylaxie des accidents thrombotiques artériels (par exemple, chez les patients porteurs de valves cardiaques artificielles, dans la prévention des accidents vasculaires cérébraux, dans la prévention de l'infarctus du myocarde). Ils pourront être associés aux produits habituellement employés tels que l'héparine ou les fibrinolytiques dans le traitement des thromboses aigues.

Les doses et schémas thérapeutiques seront fonction du sujet et de l'affection à traiter. Les produits pourront être administrés par voie orale (par exemple sous forme de gélules, comprimés, gouttes buvables), par voie injectable (soluté injectable par voie intramusculaire ou intraveineuse; administration en perfusion intraveineuse), par voie rectale (suppositoires), par voie locale (collyres pour le traitement du glaucome, aérosols pour le traitement de la crise d'asthme). Suivant les indications la dose quotidienne variera de 1 à 100 mg en une à trois prises pour la voie orale, de 1 à 100 mg en une ou deux prises pour la voie rectale; la dose administrée par voie intraveineuse pourra varier entre 0,1 et 10 mg. Les collyres contiendront 0,05 à 0,5% de principe actif et les aérosols délivreront de 0,1 à 10 mg de principe actif par inhalation.

**Revendications**

1. Nouveaux produits de formule générale

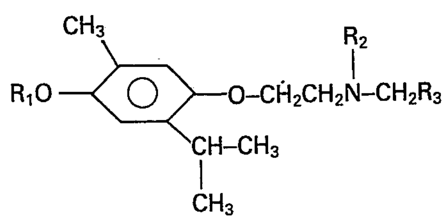

dans laquelle

$R_1 = H, CH_3, COCH_3$
$R_2 = H, CH_3$

$R_3$ = 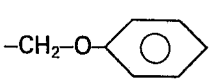

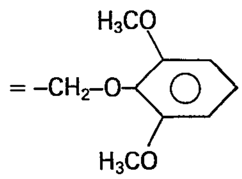

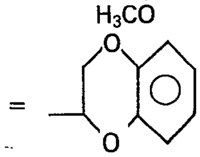

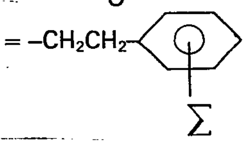

avec Σ = H, ou un ou plusieurs substituants choisis parmi halogène, OH et $OCH_3$.

2. Nouveaux produits selon la revendication 1 tels que

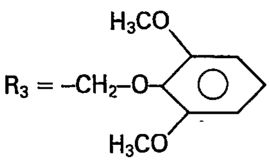

ou

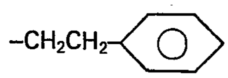

3. Méthode de préparation des produits selon les revendications 1 et 2 caractérisée en ce que l'isopropyl-5 méthyl-2 N-méthylaminoéthoxy-4 phénol réagit avec le chlorure de l'acide $R_3COOH$ dans lequel

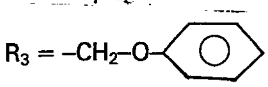

,

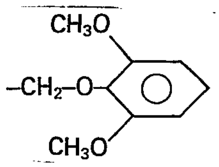

,

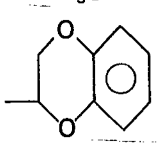

ou

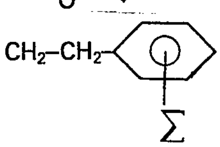

avec Σ = H ou un ou plusieurs substituants tels que OH, halogène, $OCH_3$ pour former

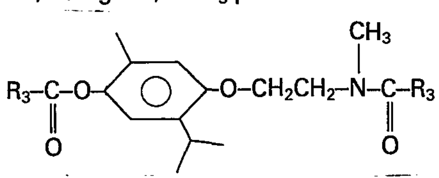

et en ce que ce produit est réduit par $LiA1H_4$.

4. Nouveaux médicaments contenant une quantité thérapeutiquement efficace d'au moins un produit selon les revendications 1 et 2.

**Patentansprüche**

1. Neue Verbindungen der allgemeinen Formel

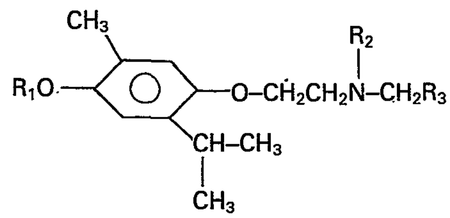

in der

$R_1 = H, CH_3, COCH_3$
$R_2 = H, CH_3$

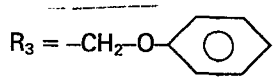

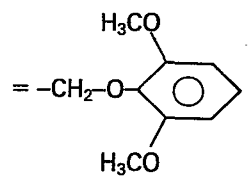

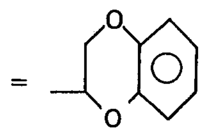

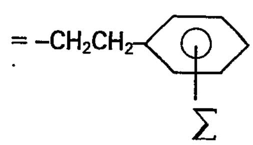

sind, wobei Σ = H oder ein Substituent oder mehrere aus der Gruppe Halogen, OH und $OCH_3$ ist.

2. Verbindungen nach Anspruch 1, in denen

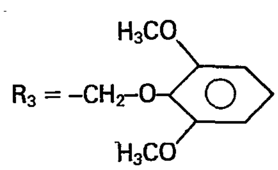

oder

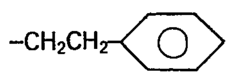

ist.

3. Verfahren zur Herstellung von Verbindungen nach Anspruch 1 oder 2, dadurch gekennzeichnet, dass man Isopropyl-5-methyl-2-N-methylamino-ethoxy-4-phenol mit einem Säurechlorid der Säure der Formel $R_3COOH$, in der

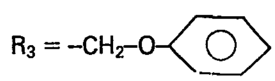

,

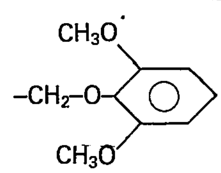

,

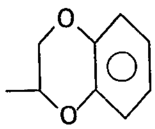

oder

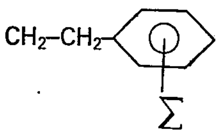

bedeutet, wobei Σ ein Substituent oder mehrere wie OH, Halogen oder $OCH_3$ ist, zu einer Verbindung der Formel

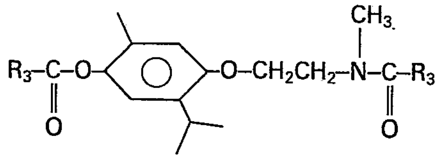

und diese Verbindung mit $LiA1H_4$ reduziert.

4. Arzneimittel, enthaltend mindestens eine der Verbindungen nach Anspruch 1 oder 2 in einer therapeutisch wirksamen Menge.

**Claims**

1. New products having the general formula:

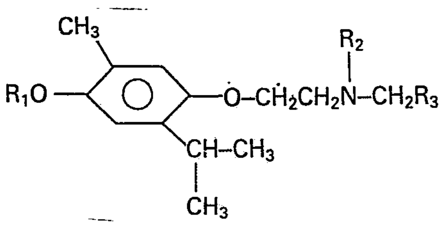

wherein

$R_1$ = H, $CH_3$, $COCH_3$
$R_2$ = H, $CH_3$

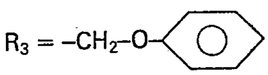

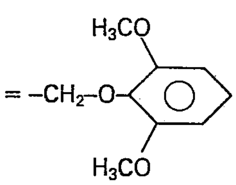

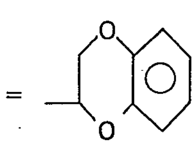

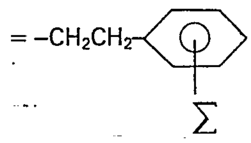

in which Σ represents H or one or more substituents from the groups comprising halogens, OH, and $OCH_3$.

2. New products according to claim 1, such as

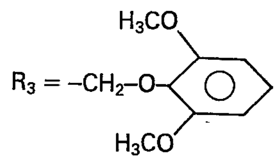

or

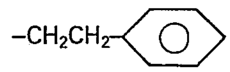

3. Process for preparing products according to claims 1 ans 2, characterized in that 5-isopropyl-2-methyl-4-N-methylaminoethoxyphenol is reacted with acid chloride $R_3COOH$ wherein

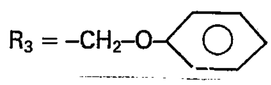

,

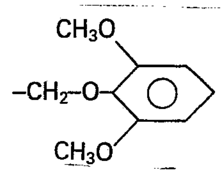

,

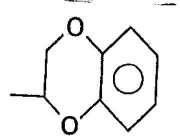

or

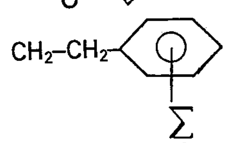

in which is hydrogen or one or more substituents from the group comprising OH, halogens and $OCH_3$ in order to obtain

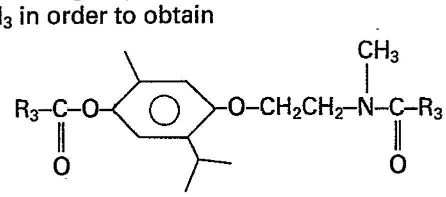

and this product is reacted with $LiA1H_4$.

4. New medecines comprising a therapeutically effective amount of at least one product according to claims 1 and 2.